# EUROPEAN PATENT SPECIFICATION

(11) **EP 1 455 854 B1**
(45) Date of publication and mention of the grant of the patent: **25.05.2005**
(21) Application number: 02804895.7
(22) Date of filing: 13.12.2002
(51) Int. Cl.: A61L 27/38, A61L 27/24, A61L 27/60, A61F 2/10, C12N 5/06

(54) **SKIN/HAIR EQUIVALENT WITH RECONSTRUCTED PAPILLAE**
HAUT/HAAR-ÄQUIVALENT MIT REKONSTRUIERTEN PAPILLEN
EQUIVALENT DERMIQUE/CAPILLAIRE AVEC PAPILLE RECONSTRUITE

(30) Priority: 19.12.2001 DE 10162814
(43) Date of publication of application: 15.09.2004
(73) Proprietor: Henkel Kommanditgesellschaft auf Aktien, 40589 Düsseldorf (DE); Cutech S.R.L., 35127 Camin, Padova (IT)
(72) Inventor: SCHLOTMANN, Kordula, 40627 Düsseldorf (DE); GASSENMEIER, Thomas, 40229 Düsseldorf (DE); PAUS, Ralf, 22453 Hamburg (DE); GIESEN, Melanie, 47608 Geldern (DE); PETERSOHN, Dirk, 50996 Köln (DE)
(86) International application number: PCT/EP2002/014212
(87) International publication number: WO 2003/051419

(56) References cited:
- EP-A- 0 285 471
- WO-A-97/25995
- WO-A-97/41208
- WO-A-99/01034
- US-A- 4 919 664
- US-A- 5 639 654
- ARASE S ET AL: "CO-CULTURE OF HUMAN HAIR FOLLICLES AND DERMAL PAPILLAE IN A COLLAGEN MATRIX" JOURNAL OF DERMATOLOGY, JAPANESE DERMATOLOGICAL ASSOCIATION, TOKYO, JP, vol. 17, no. 11, 1990, pages 667-676, XP000857988 ISSN: 0385-2407

## Description

This invention relates to a skin/hair equivalent, more particularly a skin/hair model with reconstructed papillae (pseudopapillae) in a reconstructed dermis (pseudodermis), to its production and to its use, more particularly in the medical, pharmaceutical and cosmetics fields.

Finding active substances with, for example, a biological effect on the hair follicle, so that they are capable of influencing hair pigmentation, hair growth and hair structure, requires suitable in vitro test systems on which any such effect can be evaluated. These test systems should ideally allow the screening of a relatively large number of substances, should be standardizable and inexpensive and - in the case of in vitro systems - should simulate the in vivo situation.

In hair research, there are at present no suitable in vitro models, for example for studying hair growth, hair pigmentation and hair structure. A summary of existing methods and an illustration of the disadvantages of these systems can be found, for example, in **K.S. Stenn "Laboratory Assessment of Hair Follicle Growth" in Skin Pharmacol. Appl. Skin Physiol. 1999; 12: 154-157.** These systems range from monolayer cell cultures via animal models and ex-vivo systems to in vitro systems.

Monolayer cultures of hair follicle cells have the disadvantage that, when removed from their complex three-dimensional structures, the cells behave differently than they would in the organ as a whole. Because of this, information on the effect of substances on hair follicle cells cultivated as a monolayer is of little relevance to the in vivo situation. Under the guidelines on cosmetics, animal models may not be used for the development of cosmetic products. Accordingly, ex vivo models which combine in vitro methods with in vivo methods on the animal are also out of the question. Similar problems as to the availability of material and standardizability are involved in the use of skin explantates with hairs in culture. Although in vivo studies on human beings are carried out to screen the effect, they are not advisable until a potent active substance has been discovered and incorporated in a formulation because such studies are correspondingly expensive and complex. There are also no suitable in vitro test systems for screening substances which influence hair color.

In vitro tests for modifying hair pigmentation by influencing the melanin production of the melanocytes are also mainly carried out on single cell cultures. Epidermal melanocytes or B16 melanoma cells are often used for this purpose and the results obtained with this cell type are extrapolated to the hair melanocytes because hair melanocytes are difficult to isolate and cultivate. In addition, the complex interaction of the melanocytes with the hair follicle is missing in these systems so that their relevance to the situation in vivo on the hair follicle has to be called into question. The same applies to reconstructed hair models which contain (epidermal) melanocytes. Animal models which are also a popular test model for substances with an effect on hair pigmentation are prohibited under the guidelines on cosmetics where the substances are to be used for cosmetic products. In vivo studies on human beings are laborious and expensive and, accordingly, are only advisable after a potent active substance has been found.

In the technique of tissue engineering, various cell types are isolated from tissue, for example skin tissue, and multiplied in cell culture as a so-called monolayer. The tissue is then reconstructed from the single cells. In the case of skin, fibroblasts, for example, can be "sown" into a collagen gel or other matrix so that they proliferate and form a pseudodermis. Epidermal keratinocytes can be applied to the pseudodermis thus formed where they also proliferate and form a pseudoepidermis. By raising the culture into the air (air/liquid interface), the cells begin to differentiate and to form a stratum corneum.

Hitherto, cell cultures, for example keratinocytes of the outer root sheath (ORS keratinocytes, ORS = outer root sheath), dermal papilla cells, etc., have mainly been used in hair research. However, repeated attempts have also been made to cultivate hair follicles or parts of hair follicles as a three-dimensional model, for example in a collagen gel, or to reconstruct entire hair follicles by combining different hair follicle cells.

In **"Characterization of a new tissue-engineered human skin equivalent with hair"** published in **In Vitro Cell. Dev. Biol.** - **Animal 35:318-326, June 1999,** M. Michel et al. report for the first time on the insertion of a hair follicle into a reconstructed hair model for use in penetration studies. Here, the authors used whole hair follicles which had to be prepared beforehand from hair-covered skin. Apart from the limited availability of the material, standardizability is poor where prepared hairs are used because the biological variations are considerable.

**EP 0 285 471 A1** and **EP 0 285 474 A1** also describe the production of an artificial skin which consists of a dermal layer of contractile cells (fibroblasts) and extracellular matrix components into which whole hair follicles or follicle segments are inserted. The dermal layer is then additionally coated with keratinocytes which form an epidermal layer. The disadvantage here is that the papillae are not reconstructed, instead only part of the hair follicle with no papilla is used.

The model used by a group of Japanese researchers **(M. Inamatsu et al. "Hair Follicle Development in Organotypic Culture", Third Intercontinental Meeting of Hair Research Societies (Abstract), Tokyo, 2001)** for the early phase of hair development consists of freshly isolated dermal papillae from rat whiskers which are inserted between a collagen gel containing fibroblasts and an epidermal layer of rat keratinocytes. This organotypic culture is cultivated at the air/liquid interface. After 7 days, the epidermis is said to thicken in the vicinity of the dermal papillae. Firstly, no human cells or papillae are used here, secondly the papillae are not reconstructed papillae but whole papillae isolated from hair follicles and thirdly the papillae are not inserted (for example injected or grafted) into the epidermis, but are placed between the dermal and the epidermal layers. The use of isolated papillae involves the same problems of availability and standardizability as the use of isolated hair follicles.

The same applies to the works of S.A.J. Watson et al. **"Sheep vibrissa dermal papillae induce hair follicle formation in heterotypic skin equivalents" in British Journal of Dermatology (1994) 131, 827-835.** Here, dermal papillae or papilla cells cultivated in a collagen gel are placed between the dermis and epidermis of a skin equivalent with a view to obtaining a model for the development of the hair follicle. Under these conditions, however, the dermal papilla cells migrate into the dermal matrix and do not form themselves into a papilla so that the model has to be modified by applying the papillae or the papilla cells to a dermal substrate and which is then covered by a fetal mouse epidermis and transplanted onto hairless mice.

In 1993, A.B. Jahoda et al. **"Dermal-Epidermal Interactions - Follicle** **Derived Cell Populations in the Study of Hair-Growth Mechanisms" in J. Invest. Dermatol. 101: 33S-38S, 1993** succeeded in producing a follicle-like structure from a combination of hair cells by first cultivating outer root sheath cells (ORS cells) in the collagen capsule of the follicle of a rat whisker and then adding a mixture of different hair follicle cells - dermal papilla cells, dermal sheath cells and matrix cells. However, they needed the stable collagen capsule of the rat whisker to stabilize the structure.

A. Limat et al. "Outer Root Sheath (ORS) cells organize into epidermoid cyst-like spheroids when cultured inside Matrigel®: a light-microscopic and immunohistological comparison between human ORS cells and interfollicular keratinocytes" in **Cell Tissue Res. (1994) 275: 169-176** also fit various hair follicle cells and skin cells together in a three-dimensional structure in order to study their interaction. They use a collagen (I) gel as base and, on it, bed a layer of Matrigel® or a mixture of basal membrane components containing various cells or cell mixtures. In the Matrigel®, the epidermal or ORS keratinocytes (= outer root sheath keratinocytes) form spheroidal, but non-follicular structures. Here, layers containing various cells are placed one above the other. However, no new structure is built up in these layers like the dermal papilla. Although Limat et al. report that ORS keratinocytes are capable of forming cystoidal structures which turn horny internally, it is not a question here of the physiological nature of hair shaft formation, but rather of the differentiation and stratum corneum formation by cystoidally arranged keratinocytes. However, hair shafts are normally formed by matrix cells which lie above the dermal papilla.

**Published Japanese patent application 10-136977** (Toyobo Co., Ltd., Japan) describes an artificial tissue and its reconstruction which comprises hair-shaft-like structures at the boundary between a layer of fibroblasts in a collagen layer and a collagen layer containing hair papilla cells. This model is said to serve as a test system for determining the compatibility and effectiveness of active substances and cosmetics. The same applies here as to the article by A. Limat et al.: in this model, dermal papillae which share the three-dimensional structure of the hair follicle are not reconstructed, instead the cells are arranged one above the other in layers and no new structure is formed. Melanocytes are apparently not used in the described model either. Effectiveness tests are mentioned as a potential application for the model. However, there is no indication of which end points are to be evaluated on the model, i.e. how the application of active substances affects the structure of the reconstructed model and what can be read into this so far as the effect of this substance on hair growth and hair structure are concerned.

The "Philpott Model" **(M.P. Philpott "Human hair growth in vitro", J. Cell. Sci. 97, 463-471, 1990),** where isolated hair follicles are kept in culture for 9 days, has the disadvantage on the one hand of significant variability between the individual follicles and hence poor standardizability and, on the other hand, poor availability of the hair follicles. Because of this, only a very limited amount of active substances can be evaluated within a fixed period. In addition, only substances and formulations which are soluble in the medium can be applied, but not water-insoluble substances or formulations, such as creams for example.

In what was virtually a further development of the Philpott Model, an attempt was also made to insert isolated hair follicle segments into reconstructed skin models (see M. Inamatsu et al. and M. Michel et al.). Although follicles, follicle segments or dermal papillae are thus in contact with the dermis, which comes closer to the in vivo situation than other known models do, the disadvantages of these systems where they are to be used for studying active substances are similar to those attending the Philpott Model (poor availability of the hair follicles, no standardizability, high cost, etc.).

Accordingly, the problem addressed by the present invention was to provide a skin/hair equivalent ("skin model"), more particularly a hair/skin model with reconstructed papillae in a reconstructed dermis, which would at least partly avoid the above-mentioned disadvantages of the prior art.

Another problem addressed by the present invention was to find or provide a skin/hair equivalent or model which would be suitable as an in vitro model system, more particularly for testing and/or evaluating active substances, more particularly on the hair follicle. Such a model would be particularly suitable for discovering and testing pharmaceutical/medical and cosmetic active principles. It would also allow in vitro evaluation of the effect of such active principles on the hair follicle, hair growth, hair pigmentation, hair structure and the like.

Accordingly, the present invention relates to a process for the production of a skin/hair equivalent, more particularly a skin/hair model with reconstructed papillae (pseudopapillae; PP) in a reconstructed dermis (pseudodermis; PD), the process comprising the following steps:
(a) providing a suitable reconstructed dermis (pseudodermis; PD) or a pseudodermis preparation;
(b) providing reconstructed papillae (pseudopapillae;PP) comprising dermal papilla cells (hair papilla cells), in a suitable matrix, more particularly gel matrix, or providing corresponding precursors of such reconstructed papillae (pseudopapillae; PP) comprising cultivated dermal papilla cells (hair papilla cells), in a suitable matrix-forming, more particularly gel-forming, medium MFM_{PP} which is capable of forming a matrix, more particularly a gel matrix, in situ, more particularly in the reconstructed dermis (pseudodermis; PD);
(c) introducing or inserting the reconstructed papillae (PP) or their precursors provided in step (b) into the pseudodermis (PD) or the pseudodermis preparation provided in step (a);
(d) optionally applying a reconstructed epidermis (pseudoepidermis; PE) or a reconstructed periderm (pseudoperiderm; PI) to the pseudodermis (PD).

Accordingly, step (a) of the process comprises providing a reconstructed dermis or pseudodermis or a pseudodermis preparation. According to the invention, any pseudodermis known from the prior art may be used providing it is suitable for the process according to the invention which means in particular that it is compatible with the other constituents of the skin/hair equivalent according to the invention. In particular, the pseudodermis (PD) used in accordance with the invention comprises cultivated contractile cells, more particularly fibroblasts, preferably dermal fibroblasts, in a suitable matrix. The matrix may be, in particular, a matrix based on collagen, preferably type I and/or type III collagen, and optionally other components. The cultivated contractile cells for the pseudodermis (PD) or the pseudodermis preparation may be obtained in known manner, for example by isolating dermal fibroblasts from human or animal skin and, after cultivation, recovering the contractile cells from the resulting monolayer cultures (for example by moderate trypsinization). A suitable nutrient medium which should be compatible with the contractile cells in particular is used for cultivating those cells. According to the invention, one example of a suitable nutrient medium is essential minimal medium MEM (**M**odified **E**agle **M**edium).

The pseudodermis (PD) may then be obtained by mixing the contractile cells recovered from monolayer cultures and optionally present in a suitable nutrient medium with a matrix-forming, more particularly gel-forming, medium MFM_{PD} which contains at least one matrix former MF_{PD}, more particularly gel former, and optionally other constituents.

In the context of the invention, the resulting mixture is referred to as the pseudodermis preparation. Depending on the concentration of the matrix-forming medium, the pseudodermis preparation forms a matrix, preferably a gel matrix, relatively quickly (higher concentration of the matrix-forming medium) or relatively slowly (lower concentration) and finally contracts, optionally with ejection of any nutrient medium present, to a pseudodermis (PD). All phases or states of the matrix forming process and the contraction process are encompassed by the term "pseudodermis preparation". Accordingly, the pseudodermis is obtained on completion of the matrix formation and contraction of the pseudodermis preparation.

The matrix former MF_{PD}, more particularly gel former, of the matrix-forming, more particularly gel-forming, medium MFM_{PD} may be in particular a matrix former based on collagen, preferably type I and/or type III collagen, and optionally other components (for example constituents of the extracellular matrix of the dermis, preferably matrix and/or scleroproteins, such as laminin).

A suitable nutrient medium, more particularly essential minimal medium MEM, and optionally other components may optionally be applied to the pseudodermis (PD) thus provided or generated.

The pseudopapillae (PP) provided in step (b) comprise cultivated dermal papilla cells (hair papilla cells), on a suitable carrier and/or in a suitable matrix. The matrix may be in particular a matrix based on collagen, more particularly type IV collagen, and optionally other components. The cultivated dermal papilla cells (hair papilla cells) may be produced in known manner, for example by isolating dermal papilla cells (hair papilla cells) from the hair follicles of human or animal skin and, after cultivation, recovering the dermal papilla cells from the resulting monolayer cultures, more particularly by moderate trypsinization. The number of passages should be small, the number of passages for the dermal papillae generally being between 1 and 10 and preferably between 1 and 3. The dermal papilla cells are cultivated in a suitable nutrient medium, the nutrient medium used being in particular essential minimal medium MEM, for example DMEM (**D**ulbecco's **M**odified **E**agle **M**edium) and/or RPMI medium (medium developed by the **R**oswell **P**ark **M**emorial **I**nstitute) and/or Chang medium, optionally together with other components, for example fetal calf serum (FCS), collagen, more particularly type I collagen, and the like. Examples of nutrient media suitable in accordance with the invention for cultivating the dermal papilla cells are any RPMI- or DMEM-based cell culture media such as, for example, RPMI 1640 (Sigma) containing 20% FCS, Chang medium (Irvine Scientific) containing 10% FCS and the like.

The pseudopapillae (PP) are then obtained by mixing the papilla cells recovered from monolayer cultures and optionally present in a suitable nutrient medium with a matrix-forming, more particularly gel-forming, medium MFM_{PP} which contains at least one matrix former MF_{PP}, more particularly gel former, and optionally other constituents; the resulting mixture forms a matrix, preferably a gel, and then contracts with ejection of the nutrient medium present, if any. The pseudopapillae (PP) can then be formed (for example by punching or cutting out) from the contracted matrix or the contracted gel.

In a particularly preferred embodiment, the pseudopapillae (PP) are produced by growing dermal papilla cells, on a suitable carrier. Cell groups already interacting can thus advantageously be produced in a manner similar to the papilla.

The carrier may be both porous and non-porous and may have no pores or small or large pores. A porous surface is preferred so that the papilla cells are better able to imitate the natural three-dimensional aggregates. A swelling carrier material (for example polysaccharide- or polypeptide-based) or a non-swelling carrier material (for example polymers) may also be used.

The carrier size (or particle size, i.e. the diameter or the greatest extent in one dimension) is preferably in the range from about 50 to 2,000 µm and more preferably in the range from 100 to 1,000 µm. Particle sizes of about 100 to 500 µm are particularly preferred because, on the one hand, handling ability in the production of the pseudopapilla and, on the other hand, a sufficiently high similarity to an actual hair papilla are guaranteed. Suitable carriers are in particular the so-called microcarriers, small globular particles on which cells grow in three-dimensional geometry.

Carriers with any three-dimensional form may be used. Spherical, conical or cylindrical and polyhedral, round or ellipsoidally flattened or polygonal (for example hexagonal) carriers are suitable, spherical carriers being particularly preferred because they are able to imitate the shape of the papilla particularly well.

The carrier may consist of inorganic and/or organic material which may be both modified and unmodified at its surface.

Surface modifications may be physical in nature, for example the coating of the carrier to prevent the adhesion of particles, for example steel particles or polymers (more particularly biopolymers, preferably polypeptides and/or polysaccharides). Surface modification with matrix-forming proteins, more particularly collagen, preferably type I, III and IV collagen, and matrix and/or scleroproteins (preferably laminin, gelatine, chitosan, glucosamines, glucosaminoglycans (GAG), heparane sulfate proteoglycans, sulfated glycoproteins, such as nidogen (more particularly entactin), tissue plasmimogen activator, Matrigel® and mixtures of the above-mentioned constituents.

However, the surface can also be modified by chemical modification. For example, various surface charges can be obtained if the surface is not already charged from the carrier material itself. Carriers positively charged by surface modification of a crosslinked dextran matrix with N,N-diethylaminoethyl groups are mentioned by way of example.

Glasses, silicones or polymer matrices, for example polystyrenes, etc., are suitable. Matrices of polysaccharides, such as dextran, or polypeptides, such as gelatine, are particularly suitable. Crosslinked polymers, such as crosslinked gelatine (especially highly crosslinked gelatine, for example Cultisphere®, Percell) or crosslinked dextran, are also particularly suitable.

The pseudopapilla may be produced simply by adding the carrier to the papilla cell culture. Incubation times of one to several days or even several weeks can be suitable, depending on the cell growth density. Thorough but not overly intensive mixing of the culture medium is preferred in order to ensure optimal cell growth on the carrier surface. For example, gentle stirring or shaking or even cultivation in fluidized bed or stirred reactors or spinner bottles are suitable.

The pseudopapilla formed from the carrier covered with papilla cells may then be embedded in the prepared pseudodermis or the pseudodermis preparation.

As described in the following, however, the pseudopapillae (PP) may also be formed in situ, more particularly in the pseudodermis (PD). The matrix-forming, more particularly gel-forming, medium MFM_{PP} contains as matrix former MF_{PP}, more particularly gel former, at least one collagen, more particularly type IV collagen, and optionally other constituents selected in particular from the group of matrix and/or scleroproteins, more particularly laminin, gelatine, chitosan, glucosamines, glucosaminoglycans (GAG), heparane sulfate proteoglycans, sulfated glycoproteins, such as nidogen (more particularly entactin), tissue plasmimogen activator and growth factors, such as tissue growth factor-beta (TGF-β), fibroblast growth factor, and other growth factors from the Engelbreth-Holm-Swarm Tumor (EHS Tumor) and/or human placenta and mixtures of the above-mentioned constituents. According to the invention, the following substances, for example, are suitable matrix materials for forming the pseudopapillae (PP): Matrigel® Basement Membrane Matrix (Matrigel®), collagen, gelatine, collagen/chitosan/GAG (GAG = glucosaminoglycan), glucosamine or any other type of matrix and mixtures of these substances. Collagen, Matrigel® and mixtures thereof are particularly preferred. Matrigel® and mixtures in a ratio of 0.1:1 to 10:1 (collagen:Matrigel®) are most particularly preferred.

As previously mentioned, the pseudopapillae (PP) may be formed from the matrix, more particularly the gel, the forming/shaping of the pseudopapillae (PP) taking place either before or after the introduction or insertion of the pseudopapillae (PP) or their precursors in step (c). The pseudopapillae formed by growing papilla cells on suitable carriers, whereby each carrier particle can be regarded as a pseudopapillae, may also be used in such a matrix in this way to produce a macroscopic pseudopapillae.

Both the matrix former MF_{PD} for forming the pseudodermis (PD) and the matrix former MF_{PP} for forming the pseudopapillae (PP) should be capable of gelling on heating, more particularly at temperatures of 20°C to 40°C, for example polymerizing in the process, and of promoting the growth and differentiation of cells. The matrix of the pseudodermis (PD) and the matrix of the pseudopapillae (PP) are generally formed in three-dimensional structures. Formation of the matrix, more particularly the gel, can be reversible or irreversible although it is preferably irreversible.

The introduction or insertion of the pseudopapillae (PP) in step (c) can be carried out in various ways:

In one embodiment, suitable cavities for accommodating the pseudopapillae (PP) are first formed, preferably in the already contracted pseudodermis (PD), more particularly by punching or pricking, and the pseudopapillae (PP), which contain cultivated papilla cells and which are so shaped that their dimensions correspond to the cavities formed in the pseudodermis (PD), are then introduced or inserted (for example by grafting) into those cavities. Punching of the pseudodermis (PD) or pricking of the pseudodermis (PD) may be carried out, for example, with a punch (for example with a diameter of 0.5 to 4 mm, preferably about 2 mm) or with a button cannula or with a conventional cannula.

Before introduction of the pseudopapillae (PP) or their precursors, the cavities formed by punching or pricking of the pseudodermis (PD) may be lined (for example by spraying), more particularly with at least one collagen, preferably type IV collagen, and/or other matrix proteins, more particularly basal membrane proteins, such as laminin. The cavities are preferably lined with mixtures of two or more of the constituents mentioned, more particularly Matrigel®. Where this procedure is adopted, the pseudopapillae (PP) are preferably introduced or inserted into the pseudodermis (PD) or the pseudodermis preparation in a number or density of 1 to 50/cm² pseudodermis (PD) and more particularly 3 to 7/cm² pseudodermis (PD).

In another embodiment, the introduction or insertion of the pseudopapillae (PP) in step (c) can be carried out by directly injecting or inserting the pseudopapillae (PP), which contain the cultivated dermal papilla cells on a suitable carrier and/or in a matrix, more particularly a gel matrix, into the pseudodermis (PD) or the pseudodermis preparation. In one particular embodiment, the introduction of the pseudopapillae into the pseudodermis preparation takes place before contraction to the pseudodermis is complete. In a particularly preferred embodiment, the introduction or insertion of the pseudopapillae, preferably the injection, can take place as soon as the pseudodermis preparation has formed the matrix (or gel), preferably before or at the beginning of the contraction phase.

Where this procedure is adopted (except where pseudopapillae formed by carriers covered with papilla cells are used), the pseudopapillae (PP) are again preferably introduced or inserted into the pseudodermis (PD) or the pseudodermis preparation in a number or density of 1 to 50/cm² pseudodermis (PD) and, more particularly, 3 to 7/cm² pseudodermis (PD).

In the injection of papilla cells growing on suitable carriers, which represent individual pseudopapillae, preferably 100 to 100,000 pseudopapillae are introduced per cm³ pseudodermis.

Alternatively, the precursors of such pseudopapillae (PP) may also be injected or inserted into the pseudodermis (PD) or the pseudodermis preparation. These precursors consist of a mixture of the cultivated dermal papilla cells and at least one matrix former MF_{PP}, as described above, so that this mixture then forms the matrix and hence the pseudopapillae (PP) in situ in the pseudodermis (PD) or the pseudodermis preparation, more particularly by gelling, i.e. in this embodiment, the pseudopapillae (PP) are formed in situ in the pseudodermis (PD) or the pseudodermis preparation.

In another particularly preferred embodiment, the introduction or embedding of the pseudopapillae (PP) in step (c) may be carried out by directly mixing the pseudopapillae (PP), which contain the cultivated dermal papilla cells on a carrier, with the contractile cells and the matrix-forming, more particularly gel-forming, medium MFM_{PD}, which contains at least one matrix former MF_{PD}, more particularly gel former, and optionally other constituents, during the preparation of the pseudodermis. This early mixing of the pseudopapillae with the pseudodermis preparation leads to a uniform skin/hair equivalent. The pseudopapilla formed by the papilla cells growing on the carriers may be introduced or inserted into the pseudodermis (PD) in a density of 5,000 to 1,000,000 PP/cm³ pseudodermis (PD) and more particularly in a density of 10,000 to 80,000 pseudopapillae (carrier particles)/cm³ pseudodermis (PD). The pseudopapilla formed by the papilla cells growing on the carriers may be introduced or inserted into the pseudodermis preparation in a density of 1,000 to 100,000 PP/cm³ pseudodermis preparation (PD) and more particularly in a density of 1,500 to 60,000 pseudopapillae (carrier particles)/cm³ pseudodermis preparation (PD).

In order realistically to adjust the in vivo system, the introduction or insertion in step (c), more particularly where punches are inserted and the pseudopapillae (PP) or their precursors are injected into the pseudodermis (PD), takes place at an angle of 30° to 90° and more particularly 40° to 60°, based on the plane of the pseudodermis (PD).

The introduction or insertion of the pseudopapillae (PP) into the pseudodermis (PD) may be carried out at regular intervals.

Before or after the introduction or insertion of the pseudopapillae (PP) or their precursors in step (c), a reconstructed epidermis (pseudoepidermis; PE) or a reconstructed periderm (pseudoperiderm; PI) may optionally be applied to the pseudodermis (PD) in step (d). The pseudoepidermis is preferably applied after the introduction or insertion of the pseudopapillae (PP) or their precursors in step (c). The pseudoepidermis (PE) or the pseudoperiderm (PI) may consist of cultivated keratinocytes, hair follicle keratinocytes (ORS and/or matrix keratinocytes), more particularly outer root sheath keratinocytes (ORS keratinocytes) and/or epidermal keratinocytes and optionally of melanocytes, more particularly outer root sheath melanocytes (ORS melanocytes) and/or epidermal melanocytes, and optionally other constituents. The keratinocytes and the melanocytes present, if any, may be applied to the pseudodermis (PD) individually in separate monolayers or multilayers or together in admixture as a monolayer or multilayer. Depending on the medium used, the outer root sheath keratinocytes (ORS keratinocytes) in particular can form a pseudoperiderm (PI). The pseudoperiderm with its periderm-like structure corresponds to the conditions in the embryonic follicle morphogenesis. This has the advantage that the natural conditions, more particularly the direct and indirect cell/cell and/or cell/matrix interactions observed in a three-dimensional geometry, can thus be investigated or influenced.

One embodiment of the process according to the invention may be carried out as follows:

To produce the model, a pseudodermis (PD) is first prepared from epidermal fibroblasts in a collagen matrix. "Holes" can then punched into the pseudodermis (PD) at a certain angle (for example 30-90°) and may then optionally be lined with basal membrane proteins (for example laminin, collagen IV, etc.) or, for example, with Matrigel®, a mixture of basal membrane proteins, or mixtures of Matrigel with collagen (more particularly with type I collagen). Reconstructed papillae (pseudopapillae; PP) are then inserted into the "holes". These reconstructed papillae may be obtained by mixing dermal papilla cells cultivated from hair follicle papillae with a gel, for example Matrigel® or collagen (particularly with collagen type I) or a mixture thereof, and gelling the mix in a small number of passages. After gelation, the pseudopapillae (PP) can be punched from the gel and inserted into the pseudodermis (PD). However, gelation may also take place in situ after introduction into the pseudodermis. Alternatively, however, the dermal papilla cells embedded in Matrigel® may also be directly injected into the pseudodermis (PD) without "holes" having been punched beforehand or the dermal papilla cells embedded in Matrigel® are introduced into the pseudodermis (PD) via a pricking channel.

Finally, a cell layer of hair follicle melanocytes and/or hair follicle keratinocytes (ORS and/or matrix keratinocytes) can be applied to the pseudodermis (PD).

In the process according to the invention, therefore, the papilla is reconstructed from cultivated dermal papilla cells on a suitable carrier and/or a suitable gel former (for example Matrigel®) by introduction or insertion (for example injection, grafting, etc.) into the pseudodermis preparation or the pseudodermis (PD).Optionally, this can also be done by some form of demarcation from the surrounding environment. The reconstructed papillae (pseudopapillae; PP) comprise a combination containing a suitable gel former (for example Matrigel®) and cultivated dermal papilla cells. According to the invention, the papillae are reconstructed, for example, by insertion of so-called pseudopapilla plugs which share the three-dimensional structure of the hair follicle into the pseudodermis (PD), for example by directly injecting dermal papilla cells embedded, for example, in Matrigel® into the reconstructed dermal compartment.

In a preferred embodiment, the pseudopapilla can also be reconstructed by growing papilla cells on a suitable carrier. The papilla cells may then either be inserted into preformed "holes" in the pseudodermis or may simply be integrated therein by pressure or directly mixed with the contractile cells and the gel former (or the pseudodermis preparation) during the production of the pseudodermis.

The pseudopapilla (PP) influences the structure of the optionally overlying pseudoepidermis (PE) or the pseudoperiderm (PI) of keratinocytes and, optionally, melanocytes. A hair-follicle-like structure is formed under these conditions. The model produced in this way may be used, for example, to study substances (pharmacological, cosmetic, etc.) for hair growth and hair structure and to study the effect of substances on hair pigmentation.

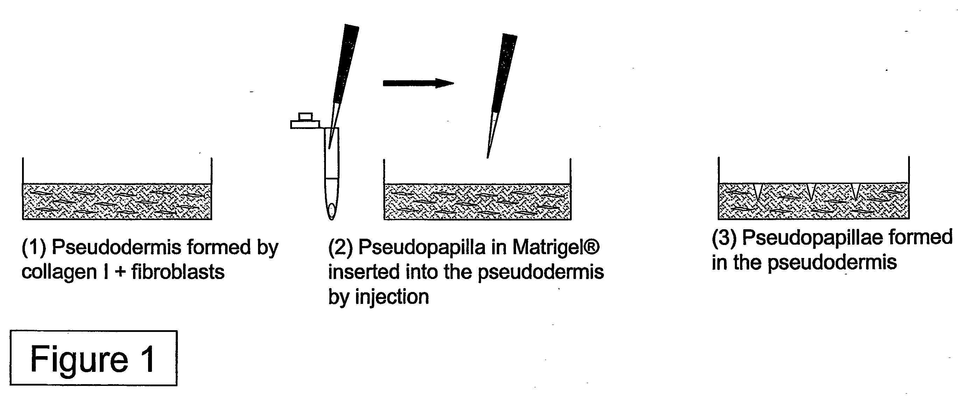

Figure 1 is a schematic illustration of one particular embodiment of the process according to the invention. A pseudodermis (PD) based on fibroblasts in a matrix of type I collagen is shown at (1). Shown at (2) is a precursor of a pseudopapilla consisting of a mixture of the cultivated dermal papilla cells and at least one matrix former MF_{PP}, from which the actual pseudopapillae (PP) can then be formed, for example by direct injection, so that this mixture then forms the matrix and hence the pseudopapillae (PP) in situ in the pseudodermis (PD), more particularly by gelling. The reconstructed papilla model shown at (3) is formed.

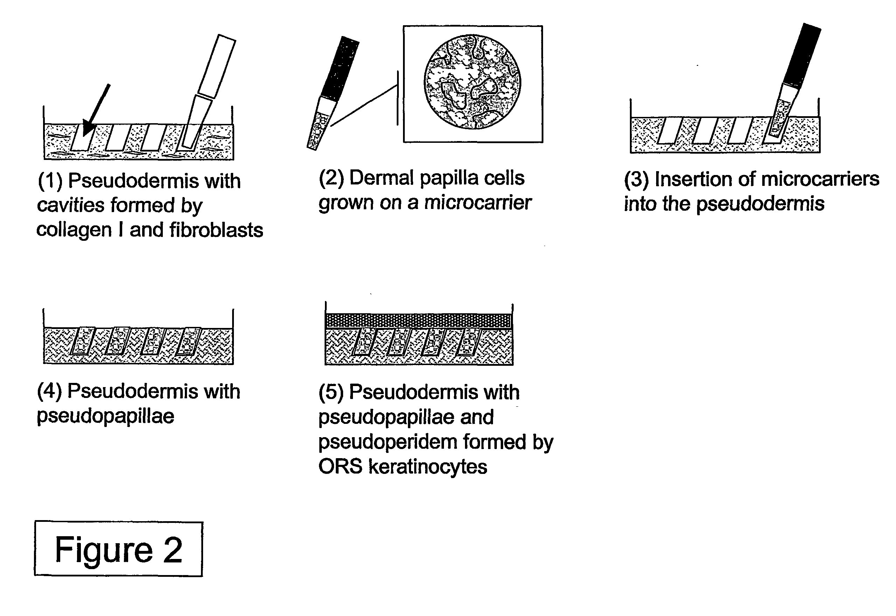

Figure 2 is a schematic illustration of another particular embodiment of the process according to the invention. Shown at (1) is a pseudodermis (PD) based on fibroblasts in a matrix of type I collagen, into which cavities or openings for accommodating the pseudopapillae (PP) have been punched, the cavities optionally being lined by spraying with type IV collagen or Matrigel®. A pseudopapilla consisting of a gelatine-based microcarrier and dermal papilla cells grown thereon is shown at (2). At (3), the pseudopapilla (PP) formed from microcarriers are introduced or inserted (for example by injection) into the pseudodermis (PD). The reconstructed papilla model shown at (4) is formed.

Follicle-like or follicular structures, including those reminiscent of the earliest stages of hair morphogenesis (morphogenesis stages I to III), such as the periderm for example, are then formed in this three-dimensional hair/skin model at (5).

The present invention also relates to the hair/skin equivalent obtainable by the process according to the invention.

The skin/hair equivalent according to the invention is in particular a skin/hair model with, in particular, three-dimensionally formed, optionally spatially demarcated, reconstructed papillae (pseudopapillae; PP) with follicle-like or follicular structures, including those reminiscent of the earliest stages of hair morphogenesis, i.e. morphogenesis stages I to III), the skin/hair equivalent comprising a reconstructed dermis (pseudodermis; PD) into which the pseudopapillae (PP) are introduced or inserted, the pseudopapillae (PP) comprising cultivated dermal papilla cells (hair papilla cells), on a suitable carrier and/or in a suitable matrix, more particularly gel matrix, and a reconstructed epidermis (pseudoepidermis; PE) or a pseudoperiderm (PI) optionally being applied to the pseudodermis (PD).

Accordingly, the skin/hair equivalent, more particularly the hair follicle model, according to the invention generally comprises a pseudodermis (PD) with dermal papillae reconstructed therein (pseudopapillae; PP). One or more layers of keratinocytes, which form or have formed themselves into a pseudoepidermis (PE) or a pseudoperiderm (PI), and optionally one or more layers of melanocytes can be applied over the pseudodermis (PD). This in vitro model is suitable, for example, for effectiveness and compatibility tests in the pharmaceutical, medical and cosmetics fields. Follicle-like or follicular structures, including those reminiscent of the earliest stages of hair morphogenesis (morphogenesis stages I to III), are formed in the three-dimensional hair/skin model according to the invention.

The present invention also relates to the use of the skin/hair equivalent according to the invention as described in claims 32 to 37. In addition, for further details, reference may be made to the foregoing observations on the process according to the invention and the skin/hair equivalent according to the invention which also apply accordingly to the use according to the invention.

The present invention also relates to a system, more particularly a test system (for example a screening system), which comprises the skin/hair equivalent according to the invention. In addition, for further details, reference may be made to the foregoing observations on the process according to the invention, the skin/hair equivalent according to the invention and the use according to the invention which also apply accordingly to the system according to the invention.

The present invention affords a number of advantages:

The skin/hair equivalent according to the invention is a reconstructed model which is more standardizable than the isolated hair follicle. It reduces the demand for hair follicles and is closer to the in vivo situation than monolayer systems. In addition, it is an alternative to animal tests.

The reconstructed model according to the invention is a complex three-dimensional model which simulates the hair follicle in vivo in its structure and its histological composition, resulting in a high level of relevance of the information provided on the effectiveness and compatibility of active substances (cosmetics, pharmaceuticals, etc.).

The following end points inter alia can be evaluated or measured to obtain information on the effectiveness of substances in regard to an improvement in hair structure and the influencing of hair growth: proliferation/apoptosis of the keratinocytes via the pseudopapilla; structure and arrangement of the keratinocytes via the pseudopapilla; structure of the epidermis; structure of the stratum comeum; volume and structure of the dermal papilla; analysis of certain hair-specific proteins (more particularly hair-specific keratins); analysis of cytokines, chemokines and all kinds of messenger substances formed inter alia by the dermal papilla; hair array analysis, proteom or expression analyses, etc.

The reconstructed hair follicle model according to the invention is the only reconstructed hair follicle model with which influences on hair pigmentation can be measured (for example pigmentation of the amelanocytic ORS melanocytes; melanin synthesis; melanin granula; arrangement of the melanocytes; migration of the melanocytes; modification of melanocyte markers, such as TRP-1, TRP-2, NKI/beteb, etc.; release of melanin to keratinocytes). It may also be used, for example, for hair array analysis.

The hair/skin model according to the invention is suitable for various applications in the medical, pharmaceutical and cosmetics fields (for example for the discovery of active substances with a biological effect on the hair follicle by influencing hair pigmentation, hair growth and hair structure, in in vitro test systems, in screening processes, for the development of cosmetic products, etc.). The model or equivalent according to the invention provides information on the effect of substances on hair follicle cells with in vivo relevance. The model or equivalent according to the invention provides hair follicles or parts of hair follicles in a three-dimensional model. In contrast to isolated hair follicles, the reconstructed papillae (pseudopapillae; PP) are available at any time and standardizable. Dermal papillae which share the three-dimensional structure of the hair follicle are reconstructed. It is possible in this way to evaluate how applied active substances act on the structure of the reconstructed three-dimensional model and what can be read into this with regard to the effect of these substances on the hair follicle (for example hair growth, hair structure, hair pigmentation, etc.).

The following Examples are intended to illustrate the invention without limiting it in any way.

### Examples

The production of a skin/hair model with reconstructed dermal papillae (pseudopapillae; PP) embedded in or inserted into a pseudodermis (PD) is described in the following. To this end, dermal papilla cells are either injected into the pseudodermis (PD), or placed by means of punches in the pseudodermis (PD). Alternatively, dermal papilla cells grown on suitable carriers can be embedded by mixing with the pseudodermis preparation. The pseudodermis (PD) may subsequently be covered with a layer of epidermal or hair follicle keratinocytes, which represent a pseudoepidermis (PE) or a pseudoperiderm (PI), and optionally with melanocytes.

The definitions defined in the following Table are used hereinafter.

### Abbreviations

| | |
|---|---|
| DMEM | Dulbecco's Modified Eagle Medium |
| DP | dermal papilla |
| DPC | dermal papilla cells |
| ECM | extracellular matrix |
| EGF | epidermal growth factor |
| FCS | fetal calf serum |
| FGF | fibroblast growth factor |
| HBSS | Hank's buffered salt solution |
| NCAM | neural cell adhesion molecule |
| NHEK | normal human epidermal keratinocytes |
| ORS keratinocytes | **o**uter **r**oot **s**heath keratinocytes |
| RPMI | RPMI Medium was developed by Moore et al. at the **R**oswell **P**ark **M**emorial **I**nstitute, hence the acronym RPMI |
| TGF | transforming growth factor |

### Procedure and methods:

### Preparation of the single cell cultures

### Dermal fibroblasts

Dermal fibroblasts are isolated from human foreskin. The epidermis and dermis of the foreskin are enzymatically separated from one another with thermolysin (0.5 mg/nl HEPES buffer). To extract the fibroblasts, the dermis is digested (3-4 h at 37°C) with collagenase H (0.2 U/ml, Boehringer Mannheim, Mannheim, Germany). After the incubation phase, the solution is carefully mixed to thin out the cells, filtered through a cell sieve and the cells are centrifuged off. Cultivation is carried out in Dulbecco's Modified Eagle Medium (DMEM) with Glutamax I (L-alanyl-L-glutamine) and sodium pyruvate, 4,500 mg L⁻¹ glucose and pyridoxine (Gibco BRL, Karlsruhe, Germany) enriched with 10% fetal calf serum (FCS) (Gibco BRL, Karlsruhe, Germany), 25 µg mL⁻¹ gentamicin (Sigma, Taufkirchen, Germany) and 100 UI mL⁻¹ penicillin G (Sigma) [**1**].

### Epidermal keratinocytes

Dermal fibroblasts are isolated from human foreskin. The epidermis and dermis of the foreskin are enzymatically separated from one another with thermolysin (0.5 mg/nl HEPES buffer). To extract the keratinocytes, the epidermis is digested (20 mins. at 37°C) with trypsin (Gibco BRL, Karlsruhe, Germany). After the incubation phase, the solution is carefully mixed to thin out the cells, filtered through a cell sieve and the cells are centrifuged off. Cultivation is carried out in a mixture of DMEM Glutamax I and Ham's F 12 (Sigma) (3:1) enriched with newborn calf serum (NCF, fetal clone II, Hyclone), epidermal growth factor (EGF) (Sigma), insulin (Sigma), hydrocortisone (Sigma), triiodo-L-thyronine (Sigma), adenine (Sigma), cholera toxin (Sigma) and antibiotics **[1]** on feeder layers (dermal fibroblasts irradiated with 60 gray to inhibit proliferation).

### Outer root sheath keratinocytes (ORS keratinocytes)

ORS keratinocytes are isolated from human hairs plucked from the back of the head. To extract the keratinocytes, the remains of the hair bulb are first removed with a scalpel and the follicles digested (40 mins. at 37°C) with trypsin (protease from Gibco BRL, Karlsruhe, Germany). After the incubation phase, the solution is carefully mixed to thin out the cells, filtered through a cell sieve and the cells are centrifuged off. Cultivation is carried out in a mixture of DMEM Glutamax I and Ham's F 12 (Sigma) (3:1) enriched with newborn calf serum (NCF, fetal clone II, Hyclone), epidermal growth factor (EGF) (Sigma), insulin (Sigma), hydrocortisone (Sigma), triiodo-L-thyronine (Sigma), adenine (Sigma), cholera toxin (Sigma) and antibiotics [**1**] on feeder layers (dermal fibroblasts irradiated with 60 gray to inhibit proliferation).

### ORS melanocytes are isolated by Tobin et al.'s method [3].

### Dermal papilla cells

Dermal papilla cells are isolated from scalp (temporal or occipital region) with intact hair follicles. To this end, the upper dermis is removed and the follicles together with the dermal papilla are plucked from the dermis using watchmaker's tweezers. The further isolation of the dermal papilla is carried out under a stereo magnifying glass. The dermal papilla is then transferred to a culture bottle coated with collagen I with the aid of a microcapillary. Cultivation is carried out either in RPMI 1640 Medium containing glutamine (Sigma) enriched with 20% fetal calf serum (FCS) (Gibco BRL, Karlsruhe, Germany) and antibiotics or in Chang's Medium containing 10% fetal calf serum [**2**].

### Production of the pseudodermis (PD)

To establish the hair model, the pseudodermis (PD) is first developed. To this end, dermal fibroblasts of the fourth passage are mixed with collagen I from rat tail tendon and sown in multiwell plates. The object of this is to optimize the number of cells, the cultivation time, the layer thickness and the size of the dermis equivalent (pseudodermis; PD). Production is carried out to the following protocol: 1 part of HBSS buffer (Gibco BRL) is mixed with 8 parts of collagen solution (Becton Dickinson) and neutralized with 1 M sodium hydroxide. The required quantity of cells is added in 1 part of fetal calf serum (FCS, Gibco BRL). The mixture obtained ( pseudodermis preparation) is poured into cell culture dishes and incubated for 1 h at 37°C in an incubation cabinet. After polymerization of the collagen, the models are covered with DMEM supplemented with 10% FCS and penicillin/streptomycin. The medium is changed three times per week over a period of seven days.

The following preparations were tested:

| Parameter | Preparation |
|---|---|
| Cell density | 1x10⁵ cells/ml gel preparation / 2.5 x 10⁵ cells/ml gel preparation |
| Cultivation time | 7 days / 14 days |
| Layer thickness of model | 4 mm; 5.2 mm; 6 mm; 8 mm; 10 mm |
| Size | 24 wells (Ø 1.6 cm), 12 wells (Ø 2.2 cm) |

### Optimizing the monolayer culture of dermal papilla cells (DPC)

To optimize the monolayer culture of dermal papilla cells, two different media are used to prepare the primary cultures.
1. RPMI 1640 (Sigma) containing 20% FCS with penicillin/streptomycin
2. Chang Medium (Irvine Scientific) containing 10% FCS

On the one hand, the primary cultures (P0) are prepared in the media shown, on the other hand a test is conducted to determine whether the medium used to continue the cultures has an effect on the proliferation and the morphology and arrangement of the cells.

### Influence of Matrigel® on the proliferation of the dermal papilla cells

Matrigel® (Becton Dickinson) may be used as the surrounding medium for the dermal papilla cells placed in the pseudodermis (PD) **[4,5].** Matrigel® is obtained from the Engelbreth-Holm-Swarm Tumor of mice and mainly contains laminin, collagen IV, heparane sulfate proteoglycans, Tissue Plasmimogen Activator, nidogen (more particularly entactin) and also TGF-β, FGF and other growth factors of the EHS Tumor. In order to study the influence of Matrigel® on the proliferation of the DPC, primary cultures of DP are carried out in cell culture bottles coated with Matrigel® and growth is observed. For comparison, DP are simultaneously grown in cell culture bottles coated with collagen I.

### Preparation of the injection channels for insertion of the DPC into the pseudodermis (PD)

To prepare the injection channels, a button cannula, a conventional cannula and a 2 mm punch are used. To show up the channels, a solution of 10% Berlin Blue in 1% agarose solution is prepared and injected into the dermis equivalent using the button cannula and the conventional cannula. Where the punches are used, the channels prepared with the biopsy punches are filled either immediately or after 24 h using the button cannula. The channels are prepared with the aid of a stereo magnifying glass. After 24 h, the models are deep-frozen to prepare cryosections and for histological examination.

### Production of the reconstructed papilla by insertion of punches into the pseudodermis (PD)

First, dermal papilla cells of the second passage were mixed in a concentration of 250,000 cells/ml with collagen I from rat tail tendon and sown in multiwell plates. The number of cells was selected in line with the production of the pseudodermis (PD).

Production was carried out to the following protocol: 1 part of HBSS buffer (Gibco BRL) was mixed with 8 parts of collagen solution (Becton Dickinson) and neutralized with 1 M sodium hydroxide. The required quantity of cells was added in 1 part of fetal calf serum (FCS, Gibco BRL). The mixture obtained was poured into cell culture dishes and incubated for 1 h at 37°C in an incubating cabinet. After polymerization of the collagen, the models were covered with Chang Medium supplemented with 10% FCS. The medium was changed three times per week over a period of eight days.

After this cultivation period, holes were made in a 7-day-old pseudodermis with the aid of a 2 mm punch. Quantities of 5 µl of Matrigel were injected and 3 mm biopsies from the polymerized collagen/DPC mixture inserted into the holes thus formed. The models were covered with Chang medium supplemented with 10% FCS and the medium was changed three times per week over a period of six days. The models were then subjected to histological and immunohistochemical evaluation.

Since collagen I in the dermal papilla is not expressed in the hair follicle and since pure Matrigel® cannot be punched, an attempt was made in another experiment to create a more physiological environment for dermal papilla cells. To this end, dermal papilla cells of the second passage were mixed in a concentration of 250,000 cells/ml with a combination of collagen I from rat tail tendon and Matrigel® and sown in multiwell plates. In the production process, the collagen normally used in the preparation of the gels was replaced by collagen/Matrigel® mixed in various ratios.

In further experiments, other punch models were prepared with an increased cell concentration (between 500,000 cells/ml and 2 x 10⁶ cells/ml) and the combination found to be optimal of 1 part Matrigel® and 2 parts collagen I. After completion, the models were subjected to histological and immunohistochemical evaluation.

### Production of the reconstructed papilla by injection of dermal papilla cells into the pseudodermis (PD) or the pseudodermis preparation

To establish very high cell densities, the cells were first mixed with Matrigel® in the required cell concentration and then centrifuged off in a refrigerated centrifuge (5 mins, 1,000 r.p.m., T = 1°C). The excess Matrigel® was then removed, the cell pellet left behind was taken up with a pipette and directly injected into the pseudodermis (PD) or the pseudodermis preparation. The models thus produced were subjected to histological and immunohistochemical evaluation.

### Production of the reconstructed papilla by embedding of microcarriers overgrown with dermal papilla cells in the pseudodermis (PD) or the pseudodermis preparation

The use of microcarriers enables dermal papilla cells to be cultivated in a predetermined, reproducible three-dimensional structure which effectively simulates the physiological constellation in the hair follicle. In principle, suitable microcarriers are any type of three-dimensional carriers on which the dermal papilla cells can be cultivated. The following carrier materials were tested:

| Microcarrier* | Specification | Manufacturer |
|---|---|---|
| Siran | Porous glass carrier, Ø 0.4-0.7 mm | Schott |
| Ashby | Porous silicone carrier with steel particles 0.8 x 0.25 mm | Ashby Scientific |
| Cultisphere S and G | Gelatine matrix, Ø 130-380 µm | Percell |
| Cytodex 1 and 3 | Dextran matrix, Ø 140-250 µm | Amersham Bioscience |
| Biosilon | Nonporous polystyrene matrix, Ø 160-300 µm | Nunc |
| 2D MicroHex | Nonporous polystyrene matrix, 125 x 25 µm | Nunc |
| Ca alginate | Nonporous material, Ø ca. 1.5 mm | Own make |

Before the microcarriers are inserted into the pseudodermis or the pseudodermis preparation, they are preincubated with dermal papilla cells so that they grow in sufficient density. Depending on the carrier selected, cultivation may be carried out in a shaken or stirred culture in a stirred or fluidized bed reactor (Eddy pro 10, Papaspyrou Biotechnologie) or in spinner bottles. The dermal papilla cells were always cultivated for several days in Chang Medium. In that time, cell growth was monitored by a neutral red coloration, MTT test, determination of the cell count and by immunohistochemical detection (for example expression of Versican) and the suitability of the various carriers was thus established.

It is also possible to produce nonporous calcium alginate beads containing dermal papilla cells. To this end, a liquid sodium alginate solution is mixed with dermal papilla cells and the resulting mixture is introduced dropwise through a cannula into a calcium chloride solution. The sodium ions are replaced by the calcium ions, resulting in a polymerization of the alginate in which the papilla cells are incorporated in the alginate.

Before insertion into the injection channels of the pseudodermis (PD) or the pseudodermis preparation (more particularly with the already formed matrix), the carriers were partly coated with Matrigel®. Instead or in addition, the channels in which the microcarriers were inserted can also be lined with Matrigel®.

Besides the injection of the microcarriers into the pseudodermis or the pseudodermis preparation with the matrix formed, the embedding of the carriers overgrown with dermal papilla cells in the pseudodermis during its production (i.e. the pseudodermis preparation) was also investigated. To this end, various volumes of microcarriers overgrown with dermal papilla cells were mixed with the dermal fibroblasts and the collagen I and the models were cultivated for 7 days.

### Production of a skin model of a pseudodermis and an epidermis of epidermal keratinocytes (NHEK) or a pseudoperiderm of hair keratinocytes (ORS keratinocytes) (with and without pseudopapilla)

In order further to develop the reconstructed hair follicle model, the pseudodermis was transferred to Snapwell Inserts (Corning Costar) after five days' culture and was first covered with epidermal keratinocytes (500,000 cells/model). After one week's submerse cultivation in keratinocyte medium (DMEM Glutamax I and Hams' F12 (Sigma) (3:1) enriched with newborn calf serum (NCF, fetal clone II, Hyclone), epidermal growth factor (EGF) (Sigma), insulin (Sigma), hydrocortisone (Sigma), triiodo-L-thyronine (Sigma), adenine (Sigma), cholera toxin (Sigma), ascorbyl-2-phosphate (Sigma) and antibiotics, the models were transferred to the air/liquid interface and cultivated for another two weeks in DMEM Glutamax I and Hams' F12 (Sigma) (3:1) enriched with insulin (Sigma), hydrocortisone (Sigma), ascorbyl-2-phosphate (Sigma), bovine serum albumin (BSA) (Sigma) and antibiotics.

In another experiment, the epidermal keratinocytes were replaced by ORS keratinocytes from the hair follicle which were sown with 800,000 cells/model. Cultivation (submerse) was carried out with keratinocyte medium for 7 days.

### Results

The following parameters were found to represent optimal conditions for the production of the pseudodermis (PD): 2.5 x 10⁵ cells/ml gel preparation (pseudodermis preparation) are cultivated for 7 days in a 12-well plate. The layer thickness at the time of sowing is 10 mm. After 7 days, the model has completely contracted, the remaining layer thickness being 3.3 mm which corresponds to a contraction of ca. 67%.

In the preparation of the DPC primary cultures, the proliferation of the cells was found to be better in Chang Medium than in RPMI Medium. When the cultures were continued, there were no differences in cell growth between the two media. Where both media are used, the cells are typically arranged in clusters both in the primary culture and in the first passage.

Where culture bottles coated with Matrigel® were used, cell growth was slower by comparison with growth surfaces coated with collagen I. This is presumably attributable to the high content of ECM molecules in Matrigel® which promotes cell differentiation in the event of slow cell growth.

A button cannula may be used to prepare the injection channels because a sufficiently large channel can be prepared with such a cannula without piercing the pseudodermis (PD). It is also possible to use punches, in which case the channels can be filled with the cell/Matrigel® mixture or punches of Matrigel® with DPC can be placed in the existing channels.

The production of the pseudopapilla by the embedding of microcarriers overgrown with dermal papilla cells by injection or by mixing with the gel preparation (or the pseudodermis preparation) is particularly suitable. Cultivation on Siran carriers in a fluidized bed reactor and, more particularly, shaking cultivation on Percell Cultispheres® can be carried out with particular advantage. Dense cell growth on the surfaces was obtained in a few days.

Coating of the pseudodermis with ORS keratinocytes in keratinocyte medium (cf. appendix) leads to the formation of a pseudoperiderm whereas coating with epidermal keratinocytes (NHEK) and keratinocytes in air/liquid interface medium leads to the formation of an epidermis.

### Appendix I: Composition of the cell culture media

### Fibroblast medium

| | |
|---|---|
| DMEM | |
| FCS | 10 % |
| Ascorbyl-2-phosphate | 1 mM |
| Penicillin G | 100 Ul/ml |
| Gentamicin | 25 µg/ml |

### Keratinocyte medium

| | |
|---|---|
| DMEM /Nutrient Mixture Ham's F12 | 3:1 |
| Fetal Clone II | 10 % |
| EGF | 10 ng/ml |
| Hydrocortisone | 0.4 µg/ml |
| Insulin | 0.12 Ul/ml |
| Cholera toxin | 10⁻¹⁰ M |
| Triiodothyronine | 2*10⁻⁹ M |
| Adenine | 24.3 µg/ml |
| Penicillin G | 100 UI/ml |
| Gentamicin | 25 µg/ml |
| Ascorbyl-2-phosphate | 1 mM |

### Papilla cell media

### RPMI-1640-Medium (Gibco, BRL, Karlsruhe)

| | |
|---|---|
| RPMI 1640 with L-glutamine | |
| FCS | 20 % |
| Penicillin | 100 UI/ml |
| Streptomycin | 100 µg/ml |

### Chang Medium (lrvine Scientific, Santa Ana, USA)

| | |
|---|---|
| Chang Medium D | |
| FCS | 10 % |

### Composition of Chang Medium D

| | |
|---|---|
| Salts | 8515 mg/ml |
| Dextrose | 880 mg/ml |
| Amino acids | 802 mg/ml |
| L-glutamine | 259 mg/ml |
| Polypeptides | 189 mg/ml |
| Vitamins | 61 mg/ml |
| Deoxyribonucleosides | 35 mg/ml |
| Ribonucleosides | 35 mg/ml |
| Sodium pyruvate | 97 mg/ml |
| Steroidal hormones | 0.0009 mg/ml |
| BSA Bovine serum proteins | 12 % |
| Other components | 9 mg/ml |

### Medium for the air/liquid interface

| | |
|---|---|
| DMEM/nutrient mixture Ham's F12 | 3:1 |
| Hydrocortisone | 0.4 µg/ml |
| Insulin | 0.12 UI/ml |
| Penicillin G | 100 Ul/ml |
| Gentamicin | 25 µg/ml |
| BSA (bovine serum proteins) | 1.6 mg/ml |
| Ascorbyl-2-phosphate | 1 mM |

### Appendix II: Literature references cited in the Example

**[1]** K. Schlotmann et al., Cosmetic Efficacy Claims In Vitro Using a 3D Human Skin Model, *Int. J. Cosmet. Sci.* 23, 310-319 (2001).
**[2]** R. Warren et al., Improved Method for the Isolation and Cultivation of Human Scalp Dermal Papilla Cells, *J. Invest. Dermatol.* **98**, 693-699 (1992).
**[3]** D.J. Tobin et al., Isolation and Long-Term Culture of Human Hair-Follicle Melanocytes, *J. Invest. Dermatol*. **104,** 86-89 (1995).
**[4]** A. Limat et al., Outer root sheath cells organize into epidermoid cyst-like spheroids when cultured in Matrigel®. *Cell Tissue Res.* **275,** 169-176 (1994).
**[5]** EP 0 218 065 A2.

## Claims

1. A process for the production of a skin/hair equivalent, more particularly a skin/hair model with reconstructed papillae (pseudopapillae; PP) in a reconstructed dermis (pseudodermis; PD), the process comprising the following steps:
(a) providing a reconstructed dermis (pseudodermis; PD) or a pseudodermis preparation;
(b) providing reconstructed papillae (pseudopapillae;PP) comprising cultivated dermal papilla cells (hair papilla cells), on a suitable carrier and/or in a suitable matrix, more particularly gel matrix, or providing corresponding precursors of such reconstructed papillae (pseudopapillae;PP) comprising cultivated dermal papilla cells, in a suitable matrix-forming, more particularly gel-forming, medium MFM_{PP} which is capable of forming a matrix, more particularly a gel matrix, in situ, more particularly in the reconstructed dermis (pseudodermis; PD);
(c) introducing or inserting the reconstructed papillae (PP) or their precursors provided in step (b) into the pseudodermis (PD) or the pseudodermis preparation provided in step (a);
(d) optionally applying a reconstructed epidermis (pseudoepidermis; PE) or a reconstructed periderm (pseudoperiderm; PI) to the pseudodermis (PD).

2. A process as claimed in claim 1, **characterized in that** the pseudodermis (PD) or the pseudodermis preparation comprises cultivated contractile cells in a suitable matrix, the matrix being a matrix based on collagen, more particularly type I and/or type III collagen, and optionally other components.

3. A process as claimed in claim 1 or 2, **characterized in that** fibroblasts, more particularly dermal fibroblasts, are used as the contractile cells for the pseudodermis (PD) or the pseudodermis preparation.

4. A process as claimed in any of claims 1 to 3, **characterized in that** the cultivated contractile cells for the pseudodermis (PD) or the pseudodermis preparation are obtained by isolating dermal fibroblasts from human or animal skin and, after cultivation, recovering the contractile cells from the resulting monolayer cultures, more particularly by moderate trypsinization.

5. A process as claimed in claim 4, **characterized in that** the contractile cells, more particularly fibroblasts, preferably dermal fibroblasts, are cultivated in a suitable nutrient medium, more particularly using essential minimal medium MEM as the nutrient medium.

6. A process as claimed in any of claims 1 to 5, **characterized in that** the pseudodermis preparation is obtained by mixing contractile cells recovered from monolayer cultures and optionally present in a suitable nutrient medium with a matrix-forming, more particularly gel-forming, medium MFM_{PD} for the pseudodermis (PD) which contains at least one matrix former MF_{PD}, more particularly gel former, and optionally other constituents.

7. A process as claimed in claim 6, **characterized in that** the pseudodermis preparation forms a matrix, preferably a gel, and contracts to a pseudodermis (PD), more particularly with ejection of the nutrient medium present, if any.

8. A process, as claimed in claim 6 or 7, **characterized in that** the matrix-forming, more particularly gel-forming, medium MFM_{PD} comprises at least one collagen, more particularly type I and/or type III collagen, and optionally other components, more particularly constituents of the extracellular matrix of the dermis, preferably matrix and/or scleroproteins, such as laminin, as the matrix former MF_{PD}, more particularly gel former.

9. A process as claimed in any of claims 1 to 8, **characterized in that** a suitable nutrient medium, more particularly essential minimal medium MEM, and optionally other components are applied to the pseudodermis (PD).

10. A process as claimed in any of claims 1 to 9, **characterized in that** the pseudopapillae (PP) comprise cultivated papilla cells, more particularly dermal papilla cells, in a suitable matrix, the matrix being in particular a matrix based on collagen, more particularly type IV collagen, and optionally other components.

11. A process as claimed in any of claims 1 to 10, **characterized in that** the cultivated dermal papilla cells are obtained by isolating dermal papilla cells from the hair follicles of human or animal skin and, after cultivation, recovering the dermal papilla cells from the resulting monolayer cultures, more particularly by moderate trypsinization.

12. A process as claimed in any of claims 1 to 11, **characterized in that** the dermal papilla cells are cultivated in a suitable nutrient medium, more particularly using essential minimal medium (MEM), more especially DMEM and/or RPMI medium and/or Chang medium, as the nutrient medium, optionally together with other components, more particularly fetal calf serum (FCS), collagen, more particularly type I collagen, and the like.

13. A process as claimed in any of claims 1 to 12, **characterized in that** the pseudopapillae (PP) are obtained by growing papilla cells, more particularly dermal papilla cells, on a suitable carrier.

14. A process as claimed in claim 13, **characterized in that** the carrier has a size in the range from about 50 to 2,000 µm, preferably in the range from 100 to 1,000 µm and more preferably in the range from about 100 to 500 µm.

15. A process as claimed in claim 13 or 14, **characterized in that** the carrier material is selected from polysaccharides or polypeptides, more particularly gelatine or dextran, which are preferably crosslinked.

16. A process as claimed in any of claims 1 to 12, **characterized in that** the pseudopapillae (PP) are obtained by mixing papilla cells recovered from monolayer cultures and optionally present in a suitable nutrient medium with a matrix-forming, more particularly gel-forming, medium MFM_{PP} for the pseudopapillae (PP) which contains at least one matrix former MF_{PP}, more particularly gel former, and optionally other constituents, the resulting mixture forming a matrix, preferably a gel, and contracting, more particularly with ejection of the nutrient medium present, if any.

17. A process as claimed in claim 16, **characterized in that** the matrix-forming, more particularly gel-forming, medium MFM_{PP} contains as matrix former MF_{PP}, more particularly gel former, at least one collagen, more particularly type IV collagen, and optionally other constituents selected in particular from the group of matrix and/or scleroproteins, more particularly laminin, gelatine, chitosan, glucosamines, glucosaminoglycans (GAG), heparane sulfate proteoglycans, sulfated glycoproteins, such as nidogen (more particularly entactin), and growth factors, such as tissue growth factor-beta (TGF-β), fibroblast growth factor, tissue plasmimogen activator and other growth factors from the Engelbreth-Holm-Swarm Tumor (EHS Tumor) and/or human placenta and mixtures of the above-mentioned constituents.

18. A process as claimed in claim 16 or 17, **characterized in that** the contracted matrix, more particularly the gel, and/or the pseudopapillae (PP) is/are formed in situ in the pseudodermis (PD) or the pseudodermis preparation.

19. A process as claimed in any of claims 1 to 18, **characterized in that** the pseudopapillae (PP) are formed from the matrix, more particularly the gel, the forming/shaping of the pseudopapillae (PP) taking place either before or after the introduction or insertion of the pseudopapillae (PP) or their precursors in step (c).

20. A process as claimed in any of claims 1 to 19, **characterized in that** the matrix former MF_{PD} for the pseudodermis (PD) and/or the matrix former MF_{PP} for the pseudopapillae (PP) is capable of gelling on heating, more particularly at temperatures of 20°C to 40°C, more particularly polymerizing in the process, and of promoting the growth and differentiation of cells.

21. A process as claimed in any of claims 1 to 20, **characterized in that** the matrix of the pseudodermis (PD) and/or the matrix of the pseudopapillae (PP) forms a three-dimensional structure.

22. A process as claimed in any of claims 1 to 21, **characterized in that** formation of the matrix, more particularly the gel, is reversible or irreversible.

23. A process as claimed in any of claims 1 to 21, **characterized in that** the introduction or insertion of the pseudopapillae (PP) in step (c) is carried out by forming suitable cavities in the pseudodermis (PD) for accommodating the pseudopapillae (PP), more particularly by punching or pricking, and then introducing, inserting or grafting the pseudopapillae (PP), which contain cultivated papilla cells and of which the dimensions correspond to cavities thus formed in the pseudodermis (PD), into those cavities or by introducing or inserting the pseudopapillae (PP) or corresponding precursors of such pseudopapillae (PP) in the form of a mixture of cultivated dermal papilla cells and at least one matrix former MF_{PP}, the precursors of the pseudopapillae (PP) then forming the matrix in situ in the pseudodermis (PD), more particularly by gelling.

24. A process as claimed in claim 23, **characterized in that** punching or pricking is carried out with a punch, more particularly with a diameter of 0.5 to 4 mm, preferably about 2 mm, or with a button cannula or with a conventional cannula.

25. A process as claimed in claim 23 or 24, **characterized in that**, before introduction of the pseudopapillae (PP) or their precursors, the cavities are lined with at least one collagen, more particularly type IV collagen, and/or other matrix proteins, more particularly basal membrane proteins, such as laminin.

26. A process as claimed in any of claims 1 to 23, **characterized in that** the preformed pseudopapillae (PP) or the precursors of the pseudopapillae (PP) are directly injected or inserted into the pseudodermis (PD) or, more particularly, into the pseudodermis preparation.

27. A process as claimed in any of claims 1 to 26, **characterized in that** the angle of the introduction or insertion of the pseudopapillae (PP) or their precursors into the pseudodermis (PD) in step (c) is 30° to 90° and more particularly 40° to 60°, based on the plane of the pseudodermis (PD).

28. A process as claimed in any of claims 1 to 27, **characterized in that** the application of the reconstructed epidermis (pseudoepidermis; PE) or of a reconstructed periderm (pseudoperiderm; PI) optionally carried out in step (d) takes place before or after the introduction or insertion of the pseudopapillae (PP) or their precursors in step (c).

29. A process as claimed in claim 28, **characterized in that** the pseudoepidermis (PE) or the pseudoperiderm (PI) contains cultivated keratinocytes, more particularly hair follicle keratinocytes (ORS and/or matrix keratinocytes) and/or epidermal keratinocytes and optionally even melanocytes, more particularly melanocytes of the outer hair root sheath (ORS melanocytes) and/or epidermal melanocytes, and optionally other constituents.

30. A process as claimed in claim 28 or 29, **characterized in that** the keratinocytes and melanocytes are applied to the pseudodermis (PD) individually in separate monolayers or multilayers or together in admixture as a monolayer or multilayer.

31. A hair/skin equivalent, more particularly a hair model with reconstructed papillae (PP) in a pseudodermis (PD) obtainable by the process claimed in claims 1 to 30.

32. The use of the skin/hair equivalent claimed in claim 31 in the pharmaceutical, medical or cosmetics and body care fields.

33. The use of the skin/hair equivalent as claimed in claim 32 for discovering, studying and/or testing pharmaceutical or cosmetic agents, more particularly for compatibility and/or effectiveness.

34. The use claimed in claim 33 for discovering, studying and/or testing pharmaceutical or cosmetic agents for effectiveness and/or compatibility with respect to the hair follicle, more particularly with regard to hair pigmentation, hair growth, hair structure, or hair color.

35. The use of the skin/hair equivalent claimed in claim 31 for the development of pharmaceutical or cosmetic agents.

36. The use of the skin/hair equivalent claimed in claim 31 in preferably automated screening processes, more particularly for the discovery, study and/or testing of pharmaceutical or cosmetic agents.

37. The use of the skin/hair equivalent claimed in claim 31 for the in vitro evaluation of the influencing of the hair follicle, hair pigmentation, hair growth, hair structure, hair color and the like, more particularly by pharmaceutical or cosmetic agents

38. A system, more particularly a test system, comprising the hair/skin equivalent claimed in claim 31.

## Patentansprüche

1. Verfahren zur Herstellung eines Haut/Haar-Äquivalents, insbesondere eines Haut-/Haarmodells mit rekonstruierten Papillen (Pseudopapillen, PP) in einer rekonstruierten Dermis (Pseudodermis, PD), das folgende Schritte umfasst:
(a) Herstellung einer rekonstruierten Dermis (Pseudodermis, PD) oder eines Pseudodermis-Ansatzes,
(b) Bereitstellung rekonstruierter Papillen (Pseudopapillen, PP), umfassend kultivierte dermale Papillenzellen (Haarpapillenzellen) auf einem geeigneten Träger und/oder in einer geeigneten Matrix, insbesondere einer Gelmatrix, oder Bereitstellung entsprechender Vorläufer solcher rekonstruierter Papillen (Pseudopapillen, PP), umfassend kultivierte dermale Papillenzellen in einem geeigneten, Matrix bildenden, insbesondere Gel bildenden Medium MFMpp, das fähig ist, eine Matrix, insbesondere eine Gelmatrix in situ, insbesondere in der rekonstruierten Dermis (Pseudodermis, PD) zu bilden,
(c) Einbringung oder Insertion der im Schritt (b) hergestellten rekonstruierten Papillen (PP) oder deren Vorläufer in die beim Schritt (a) bereitgestellte Pseudodermis (PD) oder in den Pseudodermis-Ansatz,
(d) gegebenenfalls Aufbringung einer rekonstruierten Epidermis (Pseudoepidermis, PE) oder eines rekonstruierten Periderms (Pseudoperiderms, PI) auf die Pseudodermis (PD).

2. Verfahren nach Anspruch 1, **dadurch gekennzeichnet, dass** die Pseudodermis (PD) oder der Pseudodermis-Ansatz kultivierte kontraktile Zellen in einer geeigneten Matrix, wobei die Matrix eine auf Kollagen, insbesondere Kollagen vom Typ I und/oder Typ 111 basierende Matrix ist, und optional weitere Komponente umfasst.

3. Verfahren nach Anspruch 1 oder 2, **dadurch gekennzeichnet, dass** Fibroblasten, insbesondere dermale Fibroblasten, als kontraktile Zellen für die Pseudodermis (PD) oder den Pseudodermis-Ansatz verwendet werden.

4. Verfahren nach einem der Ansprüche 1 bis 3, **dadurch gekennzeichnet, dass** die kultivierten kontraktilen Zellen für die Pseudodermis (PD) oder den Pseudodermis-Ansatz durch Isolierung dermaler Fibroblasten aus menschlicher oder tierischer Haut und, nach Kultivierung, durch Gewinnung der kontraktilen Zellen aus den resultierenden Einzelschichtkulturen, im Besonderen durch schonende Trypsinisierung, erhalten werden.

5. Verfahren nach Anspruch 4, **dadurch gekennzeichnet, dass** die kontraktilen Zellen, insbesondere die Fibroblasten, vorzugsweise dermale Fibroblasten, in einem geeigneten Nährmedium kultiviert werden, insbesondere unter Verwendung von essentiellem Minimalmedium MEM als Nährmedium.

6. Verfahren nach einem der Ansprüche 1 bis 5, **dadurch gekennzeichnet, dass** der Pseudodermis-Ansatz erhalten wird durch Mischen kontraktiler Zellen, die aus Einzelschichtkulturen gewonnen werden und optional in einem geeigneten Nährmedium vorliegen, mit einem Matrix bildenden, insbesondere Gel bildenden Medium MFMpD für die Pseudodermis (PD), das mindestens einen Matrixbilder MFpD, im Besonderen einen Gelbilder, und optional weitere Bestandteile umfasst.

7. Verfahren nach Anspruch 6, **dadurch gekennzeichnet, dass** der Pseudodermis-Ansatz eine Matrix, vorzugsweise ein Gel bildet und zu einer Pseudodermis (PD) kontrahiert, insbesondere unter Ausstoß des eventuell vorhandenen Nährmediums.

8. Verfahren nach Anspruch 6 oder 7, **dadurch gekennzeichnet, dass** das Matrix bildende, insbesondere Gel bildende Medium MFMpD mindestens ein Kollagen, insbesondere ein Kollagen vom Typ I und/oder Typ III, und optional weitere Komponente, insbesondere Bestandteile der extrazellulären Matrix der Dermis, vorzugsweise Matrix- und/oder Skleroproteine wie das Laminin als Matrixbilder MFpD, insbesondere Gelbilder, umfasst.

9. Verfahren nach einem der Ansprüche 1 bis 8, **dadurch gekennzeichnet, dass** ein geeignetes Nährmedium, insbesondere essentielles Minimalmedium MEM, und optional weitere Komponente auf die Pseudodermis (PD) aufgetragen werden.

10. Verfahren nach einem der Ansprüche 1 bis 9, **dadurch gekennzeichnet, dass** die Pseudopapillen (PP) kultivierte Papillenzellen, insbesondere dermale Papillenzellen, in einer geeigneten Matrix, wobei die Matrix vor allem eine auf Kollagen, insbesondere Kollagen vom Typ IV, basierende Matrix ist, und optional weitere Komponenten umfassen.

11. Verfahren nach einem der Ansprüche 1 bis 10, **dadurch gekennzeichnet, dass** die kultivierten dermalen Papillenzellen durch Isolierung dermaler Papillenzellen aus Haarfollikeln menschlicher oder tierischer Haut und, nach Kultivierung, durch Gewinnung der dermalen Papillenzellen aus den resultierenden Einzelschichtkulturen, insbesondere durch schonende Trypsinisierung, erhalten werden.

12. Verfahren nach einem der Ansprüche 1 bis 11, **dadurch gekennzeichnet, dass** die dermalen Papillenzellen in einem geeigneten Nährmedium, insbesondere unter Verwendung von essentiellem minimalen Mediums (MEM), spezieller eines DMEM- und/oder RPMI-Mediums und/oder Chang-Mediums als Nährmedium, optional zusammen mit anderen Komponenten, insbesondere mit fetalem Kälberserum (FCS), Kollagen, insbesondere vom Typ I, und dergleichen, kultiviert werden.

13. Verfahren nach einem der Ansprüche 1 bis 12, **dadurch gekennzeichnet, dass** die Pseudopapillen (PP) durch Wachstum von Papillenzellen, insbesondere dermalen Papillenzellen, auf einem geeigneten Träger gewonnen werden.

14. Verfahren nach Anspruch 13, **dadurch gekennzeichnet, dass** der Träger eine Größe im Bereich von etwa 50 bis 2000 µm, vorzugsweise im Bereich von 100 bis 1000 µm und am meisten bevorzugt im Bereich von etwa 100 bis 500 µm aufweist.

15. Verfahren nach Anspruch 13 oder 14, **dadurch gekennzeichnet, dass** das Trägermaterial aus den Polysacchariden oder Polypeptiden, insbesondere aus Gelatine oder Dextran, die vorzugsweise quervernetzt sind, ausgewählt wird.

16. Verfahren nach einem der Ansprüche 1 bis 12, **dadurch gekennzeichnet, dass** die Pseudopapillen (PP) durch Mischen von Papillenzellen, die aus Einzelschichtkulturen gewonnen werden und optional in einem geeigneten Nährmedium vorliegen, mit einem Matrix bildenden, insbesondere Gel bildenden Medium MFMpp für die Pseudopapillen (PP), das mindestens einen Matrixbilder MFpp, insbesondere Gelbilder und optional weitere Bestandteile umfasst, wobei das resultierende Gemisch eine Matrix, vorzugsweise ein Gel bildet und kontrahiert, insbesondere unter Ausstoß des eventuell vorhandenen Nährmediums.

17. Verfahren nach Anspruch 16, **dadurch gekennzeichnet, dass** das Matrix bildende, insbesondere Gel bildende Medium MFMpp als Matrixbilder MFpp, insbesondere als Gelbilder, mindestens ein Kollagen, insbesondere ein Kollagen vom Typ IV und optional weitere Bestandteile umfasst, die insbesondere aus der Gruppe der Matrix- und/oder Skleroproteine ausgewählt sind, insbesondere aus Laminin, Gelatine, Chitosan, Glukosaminen, Glukosaminoglykanen (GAG), Heparansulfatproteoglykanen, sulfatierten Glykoproteinen wie Nidogen (spezieller Entaktin) und Wachstumsfaktoren wie dem Gewebewachstumsfaktor Beta (TGF-β), dem Fibrolast-wachstumsfaktor, Gewebeplasminogenaktivator und anderen Wachstumsfaktoren aus dem Engelbreth-Holm-Swarm-Tumor (EHS-Tumor) und/oder der humanen Plazenta sowie Mischungen aus den oben genannten Bestandteilen.

18. Verfahren nach Anspruch 16 oder 17, **dadurch gekennzeichnet, dass** die kontrahierte Matrix, insbesondere das Gel, und/oder die Pseudopapillen (PP) in situ in der Pseudodermis (PD) oder im Pseudodermis-Ansatz gebildet werden.

19. Verfahren nach einem der Ansprüche 1 bis 18, **dadurch gekennzeichnet, dass** die Pseudopapillen (PP) aus der Matrix, insbesondere dem Gel, gebildet werden, wobei das Bilden/Formen der Pseudopapillen (PP) entweder vor oder nach Einbringung oder Insertion der Pseudopapillen (PP) oder ihrer Vorläufer beim Schritt (c) stattfindet.

20. Verfahren nach einem der Ansprüche 1 bis 19, **dadurch gekennzeichnet, dass** der Matrixbilder MFpD für die Pseudodermis (PD) und/oder der Matrixbilder MFpp für die Pseudopapillen (PP) fähig sind/ist, beim Erhitzen, insbesondere bei Temperaturen von 200C bis 40OC, zu gelieren, insbesondere bei dem Prozess zu polymerisieren, und das Wachstum und die Differenzierung von Zellen zu fördern.

21. Verfahren nach einem der Ansprüche 1 bis 20, **dadurch gekennzeichnet, dass** die Matrix der Pseudodermis (PD) und/oder die Matrix der Pseudopapillen (PP) eine dreidimensionale Struktur ausbilden/-bildet.

22. Verfahren nach einem der Ansprüche 1 bis 21, **dadurch gekennzeichnet, dass** die Bildung der Matrix, insbesondere die Gelbildung, reversibel oder irreversibel ist.

23. Verfahren nach einem der Ansprüche 1 bis 21, **dadurch gekennzeichnet, dass** die Einbringung oder Insertion der Pseudopapillen (PP) beim Schritt (c) dadurch erfolgt, dass passende Hohlräume in der Pseudodermis (PD) zur Aufnahme der Pseudopapillen (PP), insbesondere durch Stanzen oder Stechen, geformt werden und dann die Pseudopapillen (PP), die kultivierte Papillenzellen umfassen und deren Maße den auf diese Weise in der Pseudodermis (PD) gebildeten Hohlräumen entsprechen, in diese Hohlräume eingebracht, insertiert oder eingepropft werden oder dass die Pseudopapillen (PP) oder entsprechende Vorläufer solcher Pseudopapillen (PP) in Form eines Gemisches aus kultivierten dermalen Papillenzellen und mindestens einem Matrixbilder MFpp eingebracht oder insertiert werden, wobei die Vorläufer der Pseudopapillen (PP) dann die Matrix in situ in der Pseudodermis (PD), insbesondere durch Gelieren, bilden.

24. Verfahren nach Anspruch 23, **dadurch gekennzeichnet, dass** das Stanzen oder Stechen mit einer Stanze, insbesondere mit einem Durchmesser von 0,5 bis 4 mm, vorzugsweise etwa 2 mm, oder mit einer Knopfkanüle oder herkömmlichen Kanüle ausgeführt wird.

25. Verfahren nach Anspruch 23 oder 24, **dadurch gekennzeichnet, dass** vor dem Einführen der Pseudopapillen (PP) oder ihrer Vorläufer die Hohlräume mit mindestens einem Kollagen, insbesondere einem Kollagen vom Typ IV und/oder anderen Matrixproteinen, insbesondere Basalmembranproteinen wie dem Laminin, ausgekleidet werden.

26. Verfahren nach einem der Ansprüche 1 bis 23, **dadurch gekennzeichnet, dass** die vorgefertigten Pseudopapillen (PP) oder die Vorläufer der Pseudopapillen (PP) direkt in die Pseudodermis (PD) oder insbesondere in den Pseudodermis-Ansatz injiziert oder eingestochen werden.

27. Verfahren nach einem der Ansprüche 1 bis 26, **dadurch gekennzeichnet, dass** der Winkel der Einbringung oder Insertion der Pseudopapillen (PP) oder ihrer Vorläufer in die Pseudodermis (PD) beim Schritt (c) 30° bis 90° und insbesondere 40° bis 60°, bezogen auf die Ebene der Pseudodermis (PD), beträgt.

28. Verfahren nach einem der Ansprüche 1 bis 27, **dadurch gekennzeichnet, dass** die gegebenenfalls in Schritt (d) durchgeführte Aufbringung der rekonstruierten Epidermis (Pseudoepidermis, PE) oder eines rekonstruierten Periderms (Pseudoperiderms, PI) vor oder nach der im Schritt (c) durchgeführten Einbringung oder Insertion der Pseudopapillen (PP) oder ihrer Vorläufer stattfindet.

29. Verfahren nach Anspruch 28, **dadurch gekennzeichnet, dass** die Pseudoepidermis (PE) oder das Pseudoperiderm (PI) kultivierte Keratinozyten, insbesondere Haarfollikelkeratinozyten (ORS- und/oder Matrixkeratinozyten) und/oder epidermale Keratinozyten und gegebenenfalls auch Melanozyten, insbesondere Melanozyten der äußeren Haarwurzelscheide (ORS-Melanozyten) und/oder epidermale Melanozyten, und optional weitere Bestandteile umfasst.

30. Verfahren nach Anspruch 28 oder 29, **dadurch gekennzeichnet, dass** die Keratinozyten und Melanozyten einzeln in getrennten ein- oder mehrlagigen Schichten oder zusammen in Mischung als ein- oder mehrlagige Schichten auf die Pseudodermis (PD) aufgebracht werden.

31. Ein Haar/Haut-Äquivalent, insbesondere ein Haarmodell mit rekonstruierten Papillen (PP) in einer Pseudodermis (PD), erhältlich durch das Verfahren nach den Ansprüchen 1 bis 30.

32. Verwendung des Haut/Haar-Äquivalents nach Anspruch 31 im Bereich der Pharmazie, Medizin oder Kosmetik und Körperpflege.

33. Verwendung des Haut/Haar-Äquivalents nach Anspruch 32 zur Auffindung, Untersuchung und/oder Prüfung pharmazeutischer oder kosmetischer Wirkstoffe, insbesondere bezüglich deren Verträglichkeit und/oder Wirksamkeit.

34. Verwendung nach Anspruch 33 bei der Auffindung, Untersuchung und/oder Prüfung pharmazeutischer oder kosmetischer Wirkstoffe auf ihre Wirksamkeit und/oder Verträglichkeit hin im Hinblick auf den Haarfollikel, insbesondere auf die Haarpigmentierung, das Haarwachstum, die Haarstruktur oder Haarfarbe.

35. Verwendung des Haut/Haar-Äquivalents nach Anspruch 31 zur Entwicklung pharmazeutischer oder kosmetischer Wirkstoffe.

36. Verwendung des Haut/Haar-Äquivalents nach Anspruch 31 in bevorzugt automatisierten Screening-Verfahren, insbesondere zur Auffindung, Untersuchung und/oder Prüfung pharmazeutischer oder kosmetischer Wirkstoffe.

37. Verwendung des Haut/Haar-Äquivalents nach Anspruch 31 für die in vitro-Bewertung der Beeinflussung des Haarfollikels, der Haarpigmentierung, des Haarwachstums, der Haarstruktur, Haarfarbe und dergleichen, insbesondere durch pharmazeutische oder kosmetische Wirkstoffe.

38. Ein System, insbesondere ein Testsystem, das das Haar/Haut-Äquivalent nach Anspruch 31 umfasst.

## Revendications

1. Procédé pour la production d'un équivalent dermique/capillaire, plus particulièrement d'un modèle dermique/capillaire avec papilles reconstruites (pseudopapilles ; PP) dans un derme reconstruit (pseudoderme ; PD), ledit procédé comprenant les étapes suivantes :
(a) la mise à disposition d'un derme reconstruit (pseudoderme ; PD) ou d'une préparation de pseudoderme ;
(b) la mise à disposition de papilles reconstruites (pseudopapilles ; PP) comprenant des cellules de papilles dermiques cultivées (cellules de papilles capillaires), sur un support approprié et/ou dans une matrice appropriée, plus particulièrement une matrice gélifiée, ou la mise à disposition des précurseurs correspondants desdites papilles reconstruites (pseudopapilles ; PP) comprenant des cellules de papilles dermiques cultivées dans un milieu MFM_{PP} approprié pour la formation d'une matrice, plus particulièrement gélifiant, qui est capable de former une matrice, plus particulièrement une matrice gélifiée, in situ, en particulier dans le derme reconstruit (pseudoderme ; PD) ;
(c) l'introduction ou l'insertion des papilles reconstruites (PP) ou de leurs précurseurs mis à disposition dans l'étape (b) dans le pseudoderme (PD) ou la préparation de pseudoderme mise à disposition dans l'étape (a) ;
(d) éventuellement, l'application au pseudoderme (PD) d'un épiderme reconstruit (pseudo-épiderme ; PE) ou d'un périderme reconstruit (pseudopériderme ; PI).

2. Procédé selon la revendication 1, **caractérisé en ce que** le pseudoderme (PD) ou la préparation de pseudoderme comprend des cellules contractiles cultivées dans une matrice appropriée, ladite matrice étant une matrice à base de collagène, en particulier de collagène de type I et/ou de type III, et éventuellement d'autres composants.

3. Procédé selon la revendication 1 ou 2, **caractérisé en ce que** des fibroblastes, en particulier des fibroblastes dermiques, sont utilisés en tant que cellules contractiles pour le pseudoderme (PD) ou la préparation de pseudoderme.

4. Procédé selon l'une quelconque des revendications 1 à 3, **caractérisé en ce que** les cellules contractiles cultivées pour le pseudoderme (PD) ou la préparation de pseudoderme sont obtenues en isolant des fibroblastes dermiques d'une peau humaine ou animale et, après culture, en récupérant les cellules contractiles à partir des cultures en couche monocellulaire résultantes, plus particulièrement par une trypsination modérée.

5. Procédé selon la revendication 4, **caractérisé en ce que** les cellules contractiles, plus particulièrement des fibroblastes, de préférence des fibroblastes dermiques, sont cultivées dans un milieu nutritif approprié, plus particulièrement en utilisant un milieu essentiel minimum MEM comme milieu nutritif.

6. Procédé selon l'une quelconque des revendications 1 à 5, **caractérisé en ce que** la préparation de pseudoderme est obtenue en mélangeant des cellules contractiles, récupérées à partir de cultures en monocouche cellulaire et éventuellement placées dans un milieu nutritif approprié, à un milieu MFM_{PD} formant une matrice, plus particulièrement gélifiant, pour le pseudoderme (PD) qui contient au moins un formateur de matrice MF_{PD}, plus particulièrement gélifiant, et éventuellement d'autres constituants.

7. Procédé selon la revendication 6, **caractérisé en ce que** la préparation de pseudoderme forme une matrice, de préférence un gel, et se contracte en pseudoderme (PD), plus particulièrement avec éjection du milieu nutritif présent, s'il existe.

8. Procédé selon la revendication 6 ou 7, **caractérisé en ce que** le milieu MFM_{PD} de formation de matrice, plus particulièrement gélifiant, comprend au moins un collagène, plus particulièrement un collagène de type I et/ou de type III, et éventuellement d'autres composants, plus particulièrement des constituants de la matrice extracellulaire du derme, de préférence une matrice et/ou des scléroprotéines, telles que la laminine, en tant que formateur de matrice MF_{PD}, plus particulièrement gélifiant.

9. Procédé selon l'une quelconque des revendications 1 à 8, **caractérisé en ce qu'**un milieu nutritif approprié, plus particulièrement un milieu essentiel minimum MEM, et éventuellement d'autres composants sont appliqués au pseudoderme (PD).

10. Procédé selon l'une quelconque des revendications 1 à 9, **caractérisé en ce que** les pseudopapilles (PP) comprennent des cellules de papilles cultivées, plus particulièrement des cellules de papilles dermiques, dans une matrice appropriée, ladite matrice étant plus particulièrement une matrice à base de collagène, plus particulièrement de collagène de type IV, et éventuellement d'autres composants.

11. Procédé selon l'une quelconque des revendications 1 à 10, **caractérisé en ce que** les cellules de papilles dermiques cultivées sont obtenues en isolant des cellules de papilles dermiques à partir de follicules capillaires de peau humaine ou animale et, après culture, en récupérant les cellules de papilles dermiques à partir des cultures en couche monocellulaire résultantes, plus particulièrement par trypsination modérée.

12. Procédé selon l'une quelconque des revendications 1 à 11, **caractérisé en ce que** les cellules de papilles dermiques sont cultivées dans un milieu nutritif approprié, plus particulièrement en utilisant un milieu essentiel minimum (MEM), plus spécifiquement un milieu DMEM et/ou RPMI et/ou le milieu de Chang, en tant que milieu nutritif, éventuellement avec d'autres composants, plus particulièrement un sérum foetal de veau (FCS), un collagène, plus particulièrement du collagène de type I, et autres du même type.

13. Procédé selon l'une quelconque des revendications 1 à 12, **caractérisé en ce que** les pseudopapilles (PP) sont obtenues par croissance de cellules de papilles, plus particulièrement de cellules de papilles dermiques, sur un support approprié.

14. Procédé selon la revendication 13, **caractérisé en ce que** le support a une taille dans la plage d'environ 50 à 2000 µm, de préférence dans la plage de 100 à 1000 µm, et de préférence encore dans la plage d'environ 100 à 500 µm.

15. Procédé selon la revendication 13 ou 14, **caractérisé en ce que** le matériau du support est choisi parmi des polysaccharides ou des polypeptides, plus particulièrement la gélatine ou le dextrane, qui sont, de préférence, réticulés.

16. Procédé selon l'une quelconque des revendications 1 à 12, **caractérisé en ce que** les pseudopapilles (PP) sont obtenues en mélangeant des cellules de papilles, récupérées à partir de cultures en couche monocellulaire et éventuellement placées dans un milieu nutritif approprié, avec un milieu MFM_{PP} formant une matrice, plus particulièrement gélifiant, pour les pseudopapilles (PP) qui contient au moins un formateur de matrice MF_{PP}, plus particulièrement gélifiant, et éventuellement d'autres constituants, le mélange résultant formant une matrice, de préférence un gel, et se contractant, plus particulièrement avec éjection du milieu nutritif présent, s'il existe.

17. Procédé selon la revendication 16, **caractérisé en ce que** le milieu MFM_{PP} formant une matrice, plus particulièrement gélifiant, contient en tant que formateur de matrice MF_{PP}, plus particulièrement gélifiant, au moins un collagène, plus particulièrement du collagène de type IV, et éventuellement d'autres constituants choisis, en particulier, dans le groupe des matrices et/ou des scléroprotéines, plus particulièrement la laminine, la gélatine, le chitosane, des glucosamines, des glucosaminoglycanes (GAG), des protéoglycanes héparanes sulfates, des glycoprotéines sulfatées, telles que le nidogène (plus particulièrement l'entactine), et des facteurs de croissance, tels que le facteur-bêta de croissance tissulaire (TGF-β), le facteur de croissance des fibroblastes, l'activateur tissulaire du plasminogène et d'autres facteurs de croissance de Tumeur Engelbreth-Holm-Swarm (Tumeur EHS) et/ou du placenta humain et des mélanges des constituants mentionnés ci-dessus.

18. Procédé selon la revendication 16 ou 17, **caractérisé en ce que** la matrice contractée, plus particulièrement le gel, et/ou les pseudopapilles (PP) est/sont formée(s) in situ dans le pseudoderme (PD) ou la préparation de pseudoderme.

19. Procédé selon l'une quelconque des revendications 1 à 18, **caractérisé en ce que** les pseudopapilles (PP) sont formées à partir de la matrice, plus particulièrement du gel, la mise en forme/le façonnage des pseudopapilles (PP) se faisant soit avant, soit après l'introduction ou l'insertion des pseudopapilles (PP) ou de leurs précurseurs dans l'étape (c).

20. Procédé selon l'une quelconque des revendications 1 à 19, **caractérisé en ce que** le formateur de matrice MF_{PD} pour le pseudoderme (PD) et/ou le formateur de matrice MF_{PP} pour les pseudopapilles (PP) sont capables de se gélifier en chauffant, plus particulièrement à des températures de 20°C à 40°C, plus particulièrement en se polymérisant au cours du procédé, et de promouvoir la croissance et la différenciation des cellules.

21. Procédé selon l'une quelconque des revendications 1 à 20, **caractérisé en ce que** la matrice du pseudoderme (PD) et/ou la matrice des pseudopapilles (PP) forme une structure tridimensionnelle.

22. Procédé selon l'une quelconque des revendications 1 à 21, **caractérisé en ce que** la formation de la matrice, plus particulièrement du gel, est réversible ou irréversible.

23. Procédé selon l'une quelconque des revendications 1 à 21, **caractérisé en ce que** l'introduction ou l'insertion des pseudopapilles (PP) dans l'étape (c) est effectuée en formant des alvéoles appropriées dans le pseudoderme (PD) pour recevoir les pseudopapilles (PP), plus particulièrement en poinçonnant ou en piquant, puis en introduisant, insérant ou greffant les pseudopapilles (PP), qui contiennent des cellules de papilles cultivées et dont les dimensions correspondent aux alvéoles ainsi formées dans le pseudoderme (PD), dans lesdites alvéoles ou en introduisant ou insérant les pseudopapilles (PP) ou les précurseurs correspondants de ces pseudopapilles (PP) sous la forme d'un mélange de cellules de papilles dermiques cultivées et d'au moins un formateur de matrice MF_{PP}, les précurseurs des pseudopapilles (PP) formant ensuite la matrice in situ dans le pseudoderme (PD), plus particulièrement en se gélifiant.

24. Procédé selon la revendication 23, **caractérisé en ce que** le poinçonnage ou le piquage est effectué avec un poinçon, plus particulièrement d'un diamètre de 0,5 à 4 mm, de préférence d'environ 2 mm, ou avec une canule à bouton ou avec une canule conventionnelle.

25. Procédé selon la revendication 23 ou 24, **caractérisé en ce que**, avant l'introduction des pseudopapilles (PP) ou de leurs précurseurs, les alvéoles sont tapissées par au moins un collagène, plus particulièrement du collagène de type IV, et/ou d'autres protéines de matrice, plus particulièrement des protéines de membrane basale, telles que la laminine.

26. Procédé selon l'une quelconque des revendications 1 à 23, **caractérisé en ce que** les pseudopapilles (PP) préformées ou les précurseurs des pseudopapilles (PP) sont directement injectés ou insérés dans le pseudoderme (PD) ou, plus particulièrement, dans la préparation de pseudoderme.

27. Procédé selon l'une quelconque des revendications 1 à 26, **caractérisé en ce que** l'angle d'introduction ou d'insertion des pseudopapilles (PP) ou de leurs précurseurs dans le pseudoderme (PD) dans l'étape (c) est de 30° à 90° et, plus particulièrement, de 40° à 60°, en se basant sur le plan du pseudoderme (PD).

28. Procédé selon l'une quelconque des revendications 1 à 27, **caractérisé en ce que** l'application de l'épiderme reconstruit (pseudo-épiderme ; PE) ou d'un périderme reconstruit (pseudopériderme ; PI) éventuellement effectuée dans l'étape (d) a lieu avant ou après l'introduction ou l'insertion des pseudopapilles (PP) ou de leurs précurseurs dans l'étape (c).

29. Procédé selon la revendication 28, **caractérisé en ce que** le pseudo-épiderme (PE) ou le pseudopériderme (PI) contient des kératinocytes cultivés, plus particulièrement des kératinocytes de follicules capillaires (kératinocytes ORS et/ou kératinocytes de matrice) et/ou des kératinocytes épidermiques et même, éventuellement, des mélanocytes, plus particulièrement des mélanocytes de la gaine épithéliale externe capillaire (mélanocytes ORS) et/ou des mélanocytes épidermiques, et éventuellement d'autres constituants.

30. Procédé selon la revendication 28 ou 29, **caractérisé en ce que** les kératinocytes et les mélanocytes sont appliqués au pseudoderme (PD) individuellement en couches monocellulaires ou multicellulaires séparées, ou ensemble dans un mélange sous la forme d'une couche monocellulaire ou multicellulaire.

31. Equivalent capillaire/dermique, plus particulièrement modèle capillaire avec des papilles (PP) reconstruites dans un pseudoderme (PD), pouvant être obtenu par le procédé selon les revendications 1 à 30.

32. Utilisation de l'équivalent dermique/capillaire selon la revendication 31 dans les domaines pharmaceutique, médical ou cosmétique et de soins corporels.

33. Utilisation de l'équivalent dermique/capillaire selon la revendication 32 pour découvrir, étudier et/ou tester des agents pharmaceutiques ou cosmétiques, plus particulièrement pour la compatibilité et/ou l'efficacité.

34. Utilisation selon la revendication 33 pour découvrir, étudier et/ou tester des agents pharmaceutiques ou cosmétiques pour l'efficacité et/ou la compatibilité par rapport au follicule capillaire, plus particulièrement par rapport à la pigmentation capillaire, la croissance capillaire, la structure capillaire ou la coloration capillaire.

35. Utilisation de l'équivalent dermique/capillaire selon la revendication 31 pour le développement d'agents pharmaceutiques ou cosmétiques.

36. Utilisation de l'équivalent dermique/capillaire selon la revendication 31, de préférence, dans des procédés de criblage automatisés, plus particulièrement pour la découverte, l'étude et/ou le test d'agents pharmaceutiques ou cosmétiques.

37. Utilisation de l'équivalent dermique/capillaire selon la revendication 31 pour l'évaluation in vitro de l'influence plus particulièrement d'agents pharmaceutiques ou cosmétiques sur le follicule capillaire, la pigmentation capillaire, la croissance capillaire, la structure capillaire, la coloration capillaire et autres du même type.

38. Système, plus particulièrement système de test, comprenant l'équivalent capillaire/dermique selon la revendication 31.
